# EUROPÄISCHE PATENTSCHRIFT

(11) **EP 0 986 642 B1**
(45) Veröffentlichungstag und Bekanntmachung des Hinweises auf die Patenterteilung: **24.09.2003**
(21) Anmeldenummer: 98932158.3
(22) Anmeldetag: 16.06.1998
(51) Int. Cl.: C12N 15/11, A61K 31/70, C07H 21/02, C12Q 1/68

(54) **HBV-GERICHTETE ANTISENSE-NUKLEINSÄUREN**
ANTISENSE NUCLEIC ACIDS TARGETING HBV
ACIDES NUCLEIQUES ANTISENS DIRIGES CONTRE LE VIRUS DE L'HEPATITE B

(30) Priorität: 18.06.1997 DE 19725803
(43) Veröffentlichungstag der Anmeldung: 22.03.2000
(73) Patentinhaber: Patzel, Volker, Dr., 63457 Hanau (DE)
(72) Erfinder: SCZAKIEL, Georg, D-69181 Leimen (DE); PATZEL, Volker, D-63457 Hanau (DE)
(74) Vertreter: Perrey, Ralf, Dr. Dipl.-Chem.
(86) Internationale Anmeldenummer: EP9803632
(87) Internationale Veröffentlichungsnummer: WO98058055

(56) Entgegenhaltungen:
- WO-A-90/13667
- WO-A-96/03152
- WO-A-97/03211
- RITTNER K ET AL: "IN VITRO SELECTION OF FAST-HYBRIDIZING AND EFFECTIVE ANTISENSE RNASDIRECTED AGAINST THE HUMAN IMMUNODEFICIENCY VIRUS TYPE 1" NUCLEIC ACIDS RESEARCH, Bd. 21, Nr. 6, 1993, Seiten 1381-1387, XP002051228
- OH S -H ET AL: "INHIBITION OF HEPATITIS B VIRUS EXPRESSION BY ANTISENSE OLIGODEOXYRIBONUCLEOTIDES" KOREAN JOURNAL OF BIOCHEMISTRY, Bd. 25, Nr. 2, 1993, Seiten 113-118, XP000603311
- PATZEL, V. ET AL.: "Theoretical and experimental selection parameters for hepatitis B virus-directed antisense RNA are related to increased RNA-RNA annealing" BIOL. CHEM. 378(6), 539-543 , 19. Juni 1997, XP002085108
- PATZEL, V. & SCZAKIEL, G.: "Theoretical design of antisense RNA structures substantially improves annealing kinetics and efficacy in human cells." NATURE BIOTECHNOLOGY., Bd. 16, Januar 1998, Seiten 64-68, XP002085109
- SCZAKIEL, G.: "The design of antisense RNA" ANTISENSE RESEARCH AND DEVELOPMENT., Bd. 7, August 1997, Seiten 439-444, XP002085110

## Beschreibung

Die vorliegende Erfindung betrifft eine Antisense-Nukleinsäure welche gegen eine spezifische Sequenz von aus HBV stammenden RNA-Molekülen gerichtet ist und diese Antisense-Nukleinsäure enthaltende Vektoren.

Chronische Infektionen mit dem Hepatitis-B-Virus (HBV) spielen eine zentrale Rolle bei der Pathogenese von Leberzellkarzinomen beim Menschen. Hepadna-Viren, zu welchen HBV zählt, replizieren ihr DNA-Genom durch Reverse Transkription einer RNA-Zwischenstufe, der sogenannten pregenomischen RNA. Die Anwendung von Antisense-Inhibitoren stellt in besonders spezifischer Weise eine therapeutische Möglichkeit der HBV-Infektion dar. Die Vermehrung des HBV kann mit Hilfe der Antisense-Strategie sowohl durch Inhibition der Reversen Transkription, mit der pregenomischen RNA als Target, als auch durch Blockade der Translation, mit den viralen mRNAs als Targets, erfolgen. Das sinnvollste RNA-Target stellt die pregenomische RNA dar, da diese im Replikationszyklus des Virus das früheste Target für eine Antisense-Nukleinsäure ist.

Bisher wurden HBV-gerichtete Antisense-Sequenzen willkürlich insbesondere gegen 5'-Bereiche der viralen mRNAs, deren Cap-Struktur oder das Polyadenylierungssignal gerichtet (Goodarzi et al., 1990; Blum et al., 1991; Wu and Wu, 1992; Reinis et al., 1993; Yao et al., 1994; Yao et al., 1995; Moriya et al., 1996). WO 90/13367 beschreibt ein Sandwich-Hybridisierungssystem zum Nachweis von HBV in der löslichen Phase.

Da die Auswahl der Antisense-Sequenzen bislang eher willkürlich und ohne rationale Auswahlkriterien erfolgte, war ein großer Teil der untersuchten Sequenzen nicht effektiv. Wichtige Einflußgrößen auf das Assoziationsverhalten zwischen der Antisense-Nukleinsäure und der Target-RNA, wie beispielsweise die Zugänglichkeit der Target-RNAs für die Antisense-Nukleinsäuren, wurden nicht berücksichtigt. Ferner wurden bei den Antisense-RNAs die strukturelle Vielfalt nicht berücksichtigt und damit das Potential von effektiven Antisense-Inhibitoren nicht ausgeschöpft.

Somit liegt der vorliegenden Erfindung die Aufgabe zugrunde, eine Antisense-Nukleinsäure bereitzustellen, die besonders wirkungsvoll insbesondere mit der pregenomischen RNA des humanen HBV hybridisiert.

Diese Aufgabe wird durch die in den beigefügten Patentansprüchen gekennzeichneten Ausführungsformen gelöst. Insbesondere wird eine Antisense-Nukleinsäure mit der sequenz SEQ ID Nr. 1 bereitgestellt, welche gegen eine spezifische Sequenz eines von HBV stammenden RNA-Moleküls als Target bzw. Zielmolekül gerichtet ist, wobei die Antisense-Nukleinsäure kleiner als das RNA-Molekül ist und eine Assoziationskonstante k von mindestens 5 x 10³ M⁻¹ s⁻¹ aufweist. Vorzugsweise beträgt die Assoziationskonstante k mindestens 2 x 10⁴ M⁻¹ s⁻¹.

Der Begriff "Antisense-Nukleinsäure" bedeutet ein natives, halbsynthetisches, synthetisches oder modifiziertes Nukleinsäuremolekül aus Desoxyribonukleotiden und/oder Ribonukleotiden und/oder modifizierten Nukleotiden.

Das aus HBV stammende RNA-Molekül kann pregenomische RNA, preC mRNA, preS1 mRNA, preS2/S mRNA oder das Transkript des X-Gens sein. Vorzugsweise ist das RNA-Molekül die pregenomische RNA von HBV.

In der vorliegenden Erfindung enthält die Antisense-Nukleinsäure, das mit SEQ ID Nr. 1 gekennzeichnete Antisense-Oligodesoxynukleotid.

Die mit SEQ ID Nr. 1 gekennzeichnete Antisense-Nukleinsäuresequenz hybridisiert *in vitro* überdurchschnittliche schnell mit der pregenomischen RNA und ist daher ein ausgezeichneter Inhibitor der HBV-Replikation *in vivo*. Überraschenderweise wurde festgestellt, daß die bevorzugte Antisense-Nukleinsäure eine Selektivität gegenüber pregenomischer RNA von HBV aufweist, auch wenn identische Target-Sequenz-Regionen auf den mRNAs von HBV auftreten.

Ein weiterer Gegenstand der vorliegenden Erfindung ist ein Vektor, der die vorstehend definierte, erfindungsgemäße Antisense-Nukleinsäure enthält oder der eine entsprechende, zur Antisense-Nukleinsäure komplementäre DNA-Sequenz enthält, die nach einer Transkription in geeigneten Wirtszellen zur vor stehend definierten, erfindungsgemäßen Antisense-Nukleinsäure führt. Der erfindungsgemäße Vektor kann vorzugsweise geeignete regulatorische Elemente, wie Promotoren, Enhancer, Terminationssequenzen, enthalten. In einer erfindungsgemäßen Ausführungsform kann der Vektor beispielsweise zur stabilen Integration der erfindungsgemäßen Nukleinsäure in das genetische Material einer Wirtszelle verwendet werden.

Ein weiterer Gegenstand der vorliegenden Erfindung ist ein Arzneimittel, welches die erfindungsgemäße Antisense-Nukleinsäure oder den erfindungsgemäßen Vektor, gegebenenfalls in einem pharmazeutisch verträglichen Träger und/oder Verdünnungsmittel enthält. Das erfindungsgemäße Arzneimittel kann zur Hemmung oder Beseitigung von durch HBV-Infektionen verursachten Krankheitszuständen durch transiente oder stabile Integration der erfindungsgemäßen Antisense-Nukleinsäure mittels Transformation bzw. Transfektion in mit HBV infizierten Wirtszellen verwendet werden.

Die Figuren zeigen:
Figur 1 ist eine schematische Darstellung des *in vitro*-Selektionstests und der Identifizierung von schnell-hybridisierenden Antisense-RNA-Molekülen.
Figur 2 ist eine photographische Darstellung einer Autoradiographie, welche die Identifizierung von Sau5-1-abgeleiteten, schnell-hybridisierenden Antisense-RNA-Identifizierung von schnell-hybridisierenden Antisense-RNA-Molekülen.

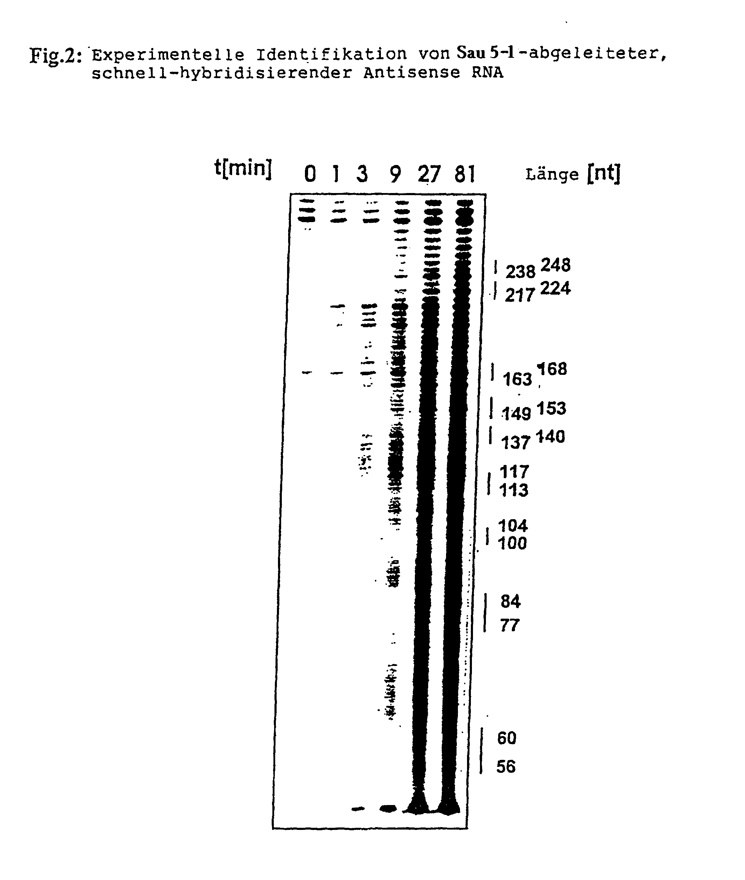

Figur 2 ist eine photographische Darstellung einer Autoradiographie, welche die Identifizierung von Sau5-1-abgeleiteten, schnell-hybridisierenden Antisense-RNA-Molekülen zeigt.

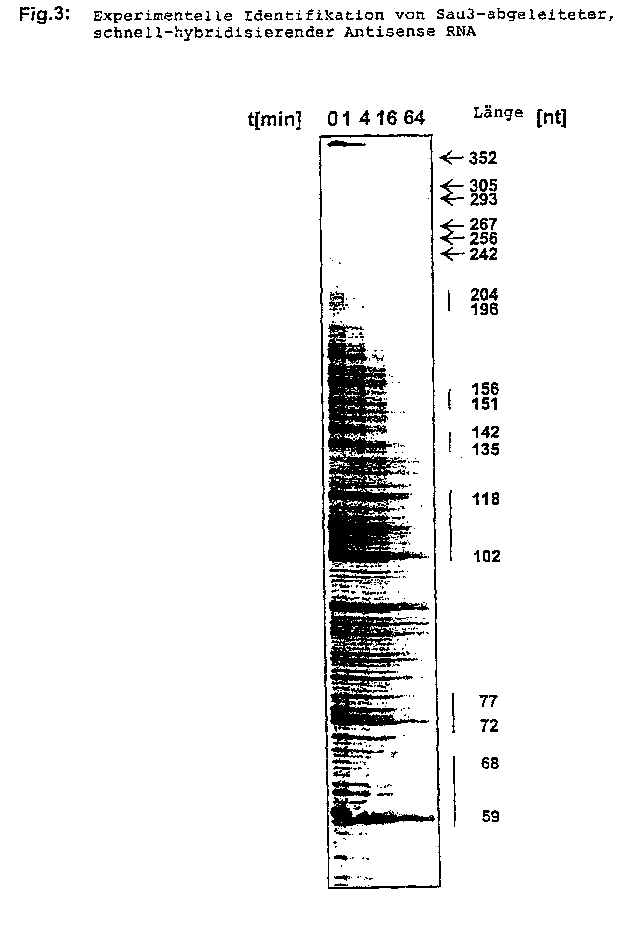

Figur 3 ist eine photographische Darstellung einer Autoradiographie, welche die Identifizierung von Sau3- abgeleiteten, schnell-hybridisierenden Antisense-RNA-Molekülen zeigt.

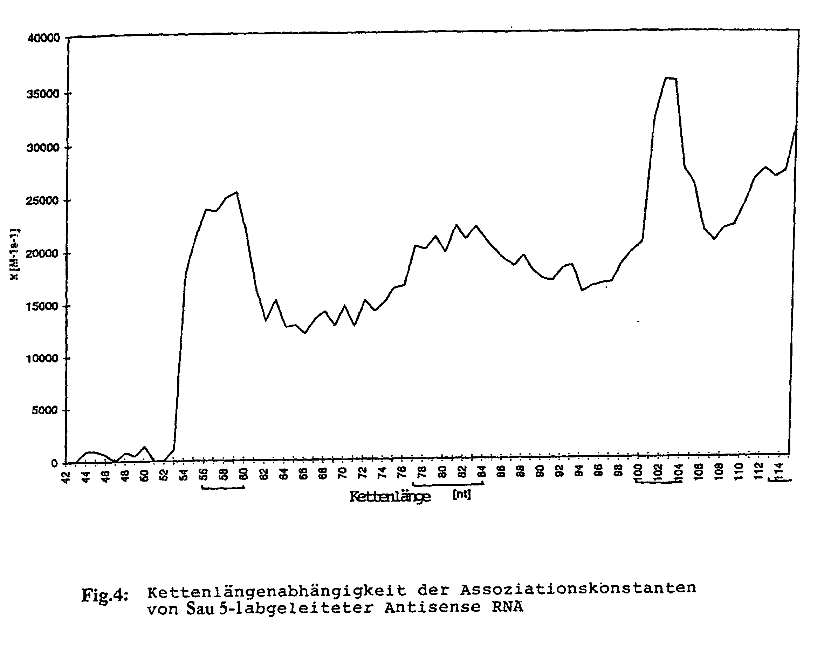

Figur 4 ist eine graphische Darstellung, welche die Kettenlängenabhängigkeit der Assoziationskonstanten von Sau5-1-abgeleiteten Antisense-RNA-Molekülen zeigt.

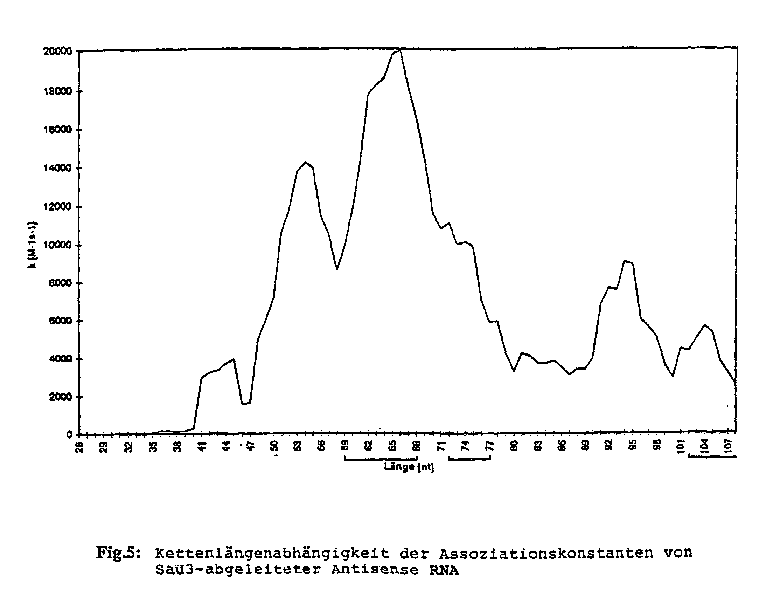

Figur 5 ist eine graphische Darstellung, welche die Kettenlängenabhängigkeit der Assoziationskonstanten von Sau3- abgeleiteten Antisense-RNA-Molekülen zeigt.

Durch das nachfolgende Beispiel wird die vorliegende Erfindung näher erläutert.

### Beispiel

### Plasmide für die in vitro Synthese von Antisense-RNAs und Target-RNA

Herkömmlich erhältliche, klonierte HBVadw2-DNA wurde mit dem Restriktionsenzym Sau3Al gespalten, und die Restriktionsfragmente wurden in die BamHI-Spaltstelle des Transkriptionsvektor pGEM-7Zf+ (Fa. Promega) kloniert und durch den DNA-Sequenzierung bestätigt. Zwei dieser Klone, pSau3 und pSau5-1, enthielten Stränge von HBVadw2 (pSau3: 334 Nukleotide, Positionen 944-1266; pSau5-1: 260 Nukleotide, Positionen 29-288) in Antisense-Orientierung hinsichtlich des SP6-Promotors. Die abgeleiteten Antisense-RNAs (Sau3 und Sau 5-1) dieser Konstrukte enthalten zusätzliche Polylinkersequenzen an ihren 5'-Enden (41 Nukleotide) und ihren 3'-Enden (6 Nukleotide). Der RNA-Strang, welcher die komplementären Sequenzen von Sau3 und Sau5-1 enthält, wurde *in* *vitro* unter Verwendung der SP6-RNA-Polymerase transkribiert. Das Sense-Transkript enthält 3320 von 3321 Nukleotiden der pregenomischen RNA (Positionen 2-3321) und zusätzliche unspezifische Sequenzen am 5'-Ende (3 Nukleotide) und am 3'-Ende (209 Nukleotide).

### In vitro-Transkription von RNA

Die Plasmide wurden vor der *in vitro*-Transkription durch folgende Enzyme linearisiert: pSau3 und pSau5-1 durch HindIII, PSP6HBV durch Pvull. SP6-RNA-Polymerase (Fa. Boehringer Mannheim) wurde zur *in vitro*-Transkription verwendet. Jeweils 5 µg linearisierte Template-RNA wurden in einem Reaktionspuffer, der 40 mM Tris-HCL, pH 8,0 (20°C), 6 mM MgCl₂, 10 mM DTT und 2 mM Spermidin, in einem Gesamtvolumen von 200 µl bei 37°C für 90 min inkubiert. Die Reaktion wurde durch Zugabe von 20 U T7 RNA-Polymerase gestartet und durch Zugabe von 10 mM MgSO₄ und 20 U DNasel (RNase-frei, Fa. Boehringer Mannheim) gestoppt. Nach einer weiteren Inkubation bei 37°C für 15 min wurde der DNasel-Abbau durch Zugabe von 25 mM EDTA gestoppt. Die RNAs wurden durch Gelfiltration (Sephadex G50-Medium, Fa. Pharmacia), Phenolextraktion und Ethanolfällung gereinigt.

### ³²P-Markierung von RNAs

Die 5'-Enden der *in vitro*-transkribierten Antisense-RNA-Molekülen (10 ng) wurden mit Phosphatase aus Kälberdarm und mit 100 µCi [γ-32P]-ATP (10,0 mCi/ml, 3000 Ci/mmol) und Polynukleotidkinase (Fa. Boehringer Mannheim) nach im Stand der Technik bekannten Methoden ³²P-markiert.

### Alkalische Hydrolyse von Antisense-RNA-Molekülen

Zur Erzeugung eines variablen Pools von HBV-gerichteten Antisense-RNA-Molekülen wurde ³²P-5'-markierte ursprüngliche RNA sukzessiv durch limitierte alkalische Hydrolyse nach im Stand der Technik bekannten Methoden gekürzt. Dabei wurden 2,5 pmol 5'-markierte RNA-Moleküle in TE-Puffer (10 mM Tris/HCl, pH 8,0, 1 mM EDTA) mit 1,5 Volumen von 0,5 mM NaHCO₃ auf 96°C für 8-10 min erhitzt, danach auf Eis abgekühlt und durch Gelfiltration (Sephadex G-50 fine, Fa. Pharmacia) entsalzt. Nach der Ethanolfällung wurden die RNA-Moleküle in TE-Puffer gelöst. Anschließend wurden die variablen Gemische von RNA-Molekülen auf 75°C für 10 min erhitzt und langsam auf 37°C vor der Verwendung in Hybridisierungstests abgekühlt, um eine native Faltung der RNA-Moleküle zu erhalten.

### Kinetische in vitro-Selektionsmethode zur selektiven Identifizierung von schnell-hybridisierenden Antisense-RNA-Molekülen

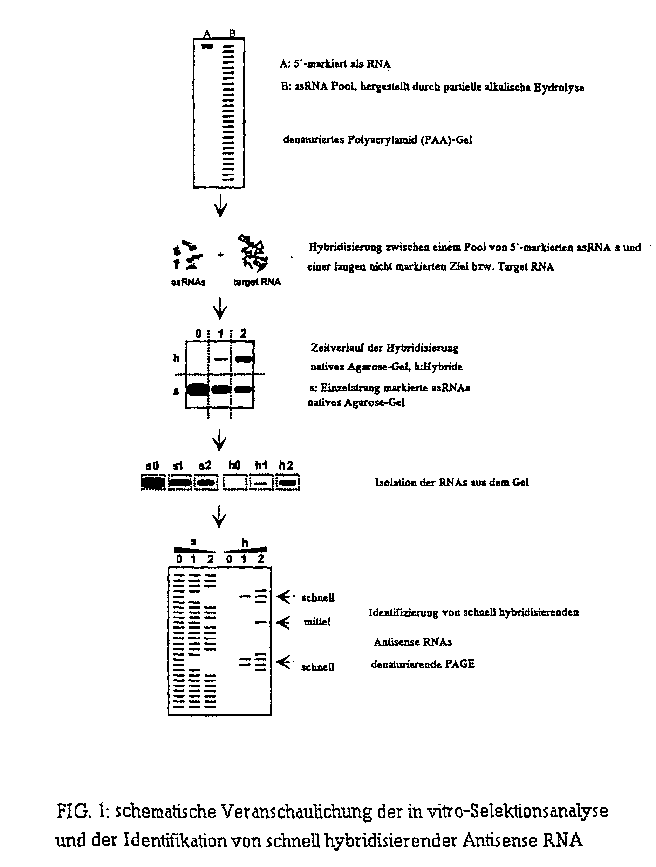

Das experimentelle Verfahren ist im Stand der Technik beschrieben und ist in Figur 1 schematisch dargestellt. 5'-markierte und teilweise hydrolisierte RNA wurde mit einem 10- oder 20-fachen molaren Überschuß (25 oder 50 pmol, Bedingungen der pseudoersten Reaktionsordnung) von HBV-pregenomischer RNA in einem Endvolumen von 60 µl und unter annähernd physiologischen Salzbedingungen (100 mM NaCI, 20 mM Tris/HCl, pH 7,4 und 10 mM MgCl₂) bei 37°C inkubiert, um die Selektion von physiologisch aktiven Molekülen zu begünstigen. Während der Hybridisierung wurden zu unterschiedlichen Zeitpunkten Aliquots des Reaktionsgemisches entnommen und in zehn Volumen vorgekühlten Stoppuffer (7M Harnstoff, 20 mM Tris/HCl, pH 8,0, 10 mM EDTA, 0,5% SDS, 0,04% Bromphenolblau, 0,04% Xylencyanol) überführt. Nach der Trennung durch native Agarose-Gelelektrophorese (0,8% Agarose, TBE) wurden die einzelsträngigen RNA-Moleküle und die gebildeten doppelsträngigen RNAs aus dem Gel geschnitten und durch Zentrifugation der Gelstücke nach "freezethaw-Methode" (flüssiger Stickstoff/37°C) wiedergewonnen. Nach der Phenolbehandlung und der Ethanolfällung wurde die RNA in Stoppuffer gelöst und Einzelstrang sowie hybridisierte Fraktionen wurden durch denaturierende PAGE (10% PAA, 7M Harnstoff, TBE) analysiert. Die Gele wurden getrocknet und einem Röntgenfilm oder einem Phosphorlmager-Screen zur weiteren computerunterstützten quantitativen Analyse exponiert.

### Bestimmung der Assoziationskonstanten von einzelnen Antisense-RNA-Molekü len

Zur quantitativen Analyse der Bandenintensitäten wurden die getrockneten PAA-Gele mittels einem Phosphorlmager (Fa. Molecular Dynamics) abgetastet. Unter Verwendung des Programms "Image Quant" (Fa. Molecular Dynamics) wurden die Bandenintensitäten gemessen und die Daten auf das Programm "EXCEL" (Fa. Microsoft) übertragen. Für einzelne RNA-Moleküle wurden die Bandenintensitäten gegen die Zeitachse aufgetragen und eine Kurve für einen einzelnen exponentiellen Zerfall wurde durch nicht-lineare Regression unter Verwendung des Programms "GRAFIT" (Fa. Erithacus Software, London, Großbritannien) angepaßt. Durch Auftragen der berechneten Assoziationskonstanten (Berechnung aufgrund von Bedingungen der pseudoersten Reaktionsordnung) gegen die Länge der Antisense-RNA-Moleküle wurden unterschiedliche Gruppen von schnell-hybridisierenden Antisense-RNA-Molekülen nachgewiesen.

### Antisense-RNA, gerichtet gegen die pregenomische RNA des menschlichen HBV vom Typ adw2

Zwei ursprüngliche Antisense-RNA-Moleküle, die gegen unterschiedliche Abschnitte der pregenomischen RNA von HBV gerichtet sind, wurden zur Durchführung eines *in* vitro-Selektionstests ausgewählt. Der erste Zielbereich, Positionen 29-288, welcher mit Abschnitten der 5'-Enden der preS1 und preS2/S mRNAs sequenzhomolog ist, war bereits als effektives Ziel bzw. Target von Antisense-Nukleinsäuren bekannt und wurde aufgrund seiner hohen Faltungsenergie der RNA-Sekundärstruktur ausgewählt. Der zweite Zielbereich, Positionen 933-1266, wurde aufgrund seiner Sequenzhomologie mit dem 5'-Ende des X-Gentranskripts ausgewählt. Die pregenomische RNA ist ungefähr 10-fach länger als diese zwei Antisense-RNA-Moleküle. Dadurch kann im Laufe des *in* vitro-Selektionstests die Einzelstrangfraktion von der Doppelstrangfraktion getrennt werden.

### Kinetische Selektion und Identifizierung von schnell-bindenden komplementären RNA

Die ursprünglichen Antisense-RNAs wurden durch *in vitro*-Transkription und ³²P-Markierung am 5'-Ende synthetisiert. Limitierte alkalische Hydrolyse hatte einen Pool von Antisense-RNA-Molekülen zur Folge, welche das 5'-Ende gemeinsam hatten, aber ein sukzessiv verkürztes 3'-Ende aufwiesen. Die Assoziationskonstanten für diese Moleküle wurde bei 37°C und physiologischer lonenstärke gemessen. Die *in vitro*-Selektion und Identifizierung von schnell-hybridisierenden Antisense-RNA-Molekülen ist in Figur 1 schematisch dargestellt. Die schnell-hybridisierenden Moleküle sind die ersten, welche von der Einzelstrangfraktion-Spur verschwinden, und durch ihre Kettenlänge nachgewiesen werden. Die experimentelle Identifizierung von schnell-bindenden, komplementären RNA-Molekülen ist in den Figuren 2 und 3 gezeigt. Die Assoziationskonstanten, bezogen auf die Kettenlänge von verwandten Molekülen, die von einem der zwei ursprünglichen Antisense-Konstrukten stammen, sind in Figuren 4 und 5 gezeigt. Die maximalen Assoziationskonstanten erreichen Werte von 2 bis 4 x 10⁴ M⁻¹ s⁻¹, und sie sind selbst für relativ kleine Antisense-RNA-Moleküle höher als für die ursprüngliche RNA mit voller Länge und 3mal höher als der Durchschnitt von allen Assoziationskonstanten von jedem der anfänglichen Pools.

### Zugänglichkeit der Target-RNA: computerunterstützte Vorhersage der Sekundärstruktur der Target-RNA

Die vollständige Sekundärstruktur der pregenomischen RNA wurde mittels des Programms "MFold" (HUSAR: Heidelberg Unix Sequence Analysis Resources) vorherbestimmt. Die Ergebnisse der *in vitro*-Selektionsexperimente stimmen mit der Zugänglichkeit der Target- bzw. Ziel-RNA überein. Damit wurden DNA-Oligonukleotidanaloga von kurzen ausgewählten Antisense-RNA-Molekülen erhalten und in transienten *in vivo*-Replikationstests untersucht.

### Liste der Sequenzen

SEQ ID Nr. 1. 5'-GCG AGG GAG TTC TTC TTC TA-3'

## Patentansprüche

1. Antisense-Nukleinsäure mit der Sequenz SEQ ID Nr.1, welche gegen eine spezifische Sequenz eines von HBV stammenden RNA-Moleküls gerichtet ist, wobei die Antisense-Nukleinsäure kleiner als das RNA-Molekül ist und eine Assoziationskonstante k von mindestens 5 x 10³ M⁻¹ s⁻¹ aufweist.

2. Vektor, enthaltend mindestens die Antisense-Nukleinsäure nach Anspruch 1.

3. Arzneimittel, enthaltend die Antisense-Nukleinsäure nach Anspruch 1 oder den Vektor nach Anspruch 2.

4. Verwendung der Antisense-Nukleinsäure nach Anspruch 1 oder des Vektors nach Anspruch 2 zur Herstellung eines Medikaments zur Hemmung oder Beseitigung von durch HBV-Infektion verursachte Krankheitszustände beim Menschen.

## Claims

1. An antisense nucleic acid having the sequence SEQ ID No.1, which is directed against a specific sequence of a RNA molecule originating from HBV, wherein the antisense nucleic acid is smaller than the RNA molecule and has an association constant k of at least 5 x 10³ M⁻¹ s⁻¹.

2. A vector containing at least the antisense nucleic acid according to claim 1.

3. A medicament containing the antisense nucleic acid according to claim 1 or the vector according to claim 2.

4. Use of the antisense nucleic acid according to claim 1 or the vector according to claim 2 for the manufacture of a medicament for inhibiting or eliminating conditions caused by HBV infection in human beings.

## Revendications

1. Acide nucléique anti-sens de séquence SEQ ID Nr1, lequel est dirigé contre une séquence spécifique d'une molécule d'ARN provenant de HBV, l'acide nucléique anti-sens étant plus petit que la molécule d'ARN et possédant une constante d'association k d'au moins 5 x 10³M⁻¹s⁻¹.

2. Vecteur contenant au moins l'acide nucléique anti-sens selon la revendication 1.

3. Produit pharmaceutique contenant l'acide nucléique anti-sens selon la revendication 1 ou le vecteur selon la revendication 2.

4. Utilisation de l'acide nucléique anti-sens selon la revendication 1 ou du vecteur selon la revendication 2 pour la préparation d'un médicament pour inhiber ou supprimer les états morbides chez l'humain provoqués par une infection par le HBV.
